# EUROPEAN PATENT SPECIFICATION

(11) **EP 1 210 086 B1**
(45) Date of publication and mention of the grant of the patent: **06.12.2006**
(21) Application number: 99938491.0
(22) Date of filing: 09.09.1999
(51) Int. Cl.: A61K 31/505, A61P 13/00

(54) **USE OF HALOFUGINONE FOR TREATING URETHRAL STRICTURE**
VERWENDUNG VON HALOFUGINONE ZUR BEHANDLUNG VON URETHRALSTRIKTUR
UTLISATION DU HALOFUGINONE DANS LE TRAITEMENT DU RETRECISSEMENT DE L'URETRE

(43) Date of publication of application: 05.06.2002
(73) Proprietor: HADASIT MEDICAL RESEARCH SERVICES & DEVELOPMENT CO., LTD., Jerusalem 91120 (IL); THE STATE OF ISRAEL-MINISTRY OF AGRICULTURE, 50250 Bet-Dagan (IL)
(72) Inventor: PINES, Mark, 76308 Rehovot (IL); VLODAVSKY, Israel, 90805 Mevasseret Zion (IL); NAGLER, Arnon, 74381 Jerusalem (IL)
(74) Representative: Grund, Martin
(86) International application number: PCT/IL1999/000441
(87) International publication number: WO 2001/017531

(56) References cited:
- WO-A-98/34616
- WO-A-98/34617
- US-A- 5 449 678
- US-A- 5 891 879
- NAGLER ARNON ET AL: "Halofuginone, an inhibitor of collagen type I synthesis, prevents postoperative adhesion formation in the rat uterine horn model" AMERICAN JOURNAL OF OBSTETRICS AND GYNECOLOGY, vol. 180, no. 3 PART 1, March 1999 (1999-03), pages 558-563, XP009023862 ISSN: 0002-9378
- NAGLER A ET AL: "HALOFUGINONE - AN INHIBITOR OF COLLAGEN TYPE I SYNTHESIS - PREVENTSPOSTOPERATIVE FORMATION OF ABDOMINAL ADHESIONS" ANNALS OF SURGERY, J. B. LIPPINCOTT COMPANY, PHILADELPHIA, US, vol. 227, no. 4, 1998, pages 575-582, XP000911972 ISSN: 0003-4932
- BASKIN L S ET AL: "Collagen analysis of urethral strictures, in vitro characterization of urethral fibroblasts and implications on the rationale for primary urethroplasties" ACTA UROLOGICA ITALICA 1995 ITALY, vol. 9, no. 4, 1995, pages 127-136, XP009024047 ISSN: 0394-2511

## Description

### FIELD AND BACKGROUND OF THE INVENTION

The present invention relates to a composition and a method for the promotion of wound heating, and in addition, to a composition and a method for the prevention of the formation of strictures, such as urethral strictures.

Wound healing is a complex process involving such factors as cells, extracellular matrix (ECM) components and the cellular microenvironment. Essentially, all wound healing involves the repair or replacement of damaged tissues. The precise nature of such repair or replacement depends upon the tissues involved, although all such processes involve certain basic principles. To illustrate these principles, cutaneous, or skin, wound healing will be described, it being understood that the discussion could also extend to all types of wound repair.

Skin has three layers, keratin, epidermis and dermis. If only the epidermis is damaged, as in most minor injuries, keratinocytes migrate from the edge of wound and eventually cover it, reforming the epidermis and keratin [D.R. Knighton and V.D. Fiegel, *Invest. Radiol.,* Vol 26, p. 604-611, 1991]. The risk of scar formation is thus relatively low for such minor injuries.

If all three skin layers are damaged or destroyed, new connective tissue, called granulation tissue, must first fill the wound space. This tissue is formed by deposition of ECM components by fibroblasts, which migrate into the wound space [D.R. Knighton and V.D. Fiegel, *Invest. Radiol.,* Vol 26, p. 604-611, 1991]. Although the formation of granulation tissue is clearly an important protective mechanism, the formation of such tissue can also lead to the formation of scars. Production of ECM components, such as collagen, has been particularly linked to scar formation. Scars on the skin can be both a cosmetic and a functional problem. For example, scar formation following serious bums can restrict the mobility of joints. Scar formation within other types of tissue, such as in the lungs after a bacterial infection, or in many organ tissues following surgery, can be extremely dangerous. One reason scars within organ tissues are so dangerous is that the scar does not duplicate the functionality of the original organ tissue, so that the healing of the wound does not lead to a complete restoration of organ capacity and function. Thus, clearly scar formation can be a pathological process.

One example of scarring which is both pathological and potentially clinically damaging is the formation of strictures, which is a common clinical condition, characterized by the narrowing of a biological passageway by a noncompliant section of scar tissue. One example of such a stricture is a urethral stricture. Such scar tissue typically arises as the reaction to an insult, which may be idiopathic in origin, as a result of instrumentation and catheterization of the urethra; the result of an external trauma; or the result of urethritis, particularly when caused by a micro-organism such as *N. gonorrhea.* The obstruction of the urethra by the stricture leads to such symptoms as hesitation for urination, weaking of the urinary stream, intermittance and the feeling of incomplete urinary evacuation. In addition, such obstruction may lead to damage to the urinary bladder, ureters and kidneys.

Strictures are generally difficult to cure or even ameliorate since the most common treatment, surgical dilatation, may be accompanied by insult to the passageway and scar tissue production, which in turn could cause luminal obstruction and treatment failure. The most successful treatment, open surgery of the passageway, is complicated and requires special training. Therefore, currently available treatments are generally unable to cure strictures.

Given the etiology of strictures, particularly with regard to the appearance of such strictures after surgery or other traumas, the involvement of fibrosis caused by excessive collagen synthesis has been suggested. For example, the content of collagen type I had been found to increase, while the content of collagen type III decreased, in urethral stricture scar formation (Baskin *et al., J. Urol.,* **157**:371, 1997). Furthermore, biopsies taken from such strictures showed dense collagen (Singh and Scott, *Br. J. Urol.,* **47**:871, 1975). Thus, clearly collagen, particular type I collagen, is an important factor in the pathology of stricture formation.

However, the deposition of ECM components, such as collagen, is currently believed to also be important for healing of the wound. Indeed, the prior art teaches that the strength of the healing wound is ultimately dependent upon collagen deposition [Haukipuro, K., *et al., Ann. Surg.,* Vol. 213, p. 75-80 , 1991]. Thus, according to the prior art, collagen deposition must be present at a sufficient level to give the healing wound strength and support, yet not at such a high level to cause the formation of scars.

If collagen deposition were prevented, permanent scars might not be formed. Therefore, these pathological processes are caused, at least in part, by the synthesis of excess collagen. Furthermore, the crucial role of collagen in other clinical conditions, such as fibrosis, has prompted attempts to develop drugs that inhibit its accumulation [K.I. Kivirikko, *Annals of Medicine,* Vol. 25, pp. 113-126 (1993)].

Such drugs can act by modulating the synthesis of the procollagen polypeptide chains, or by inhibiting specific post-translational events, which will lead either to reduced formation of extracellular collagen fibers or to an accumulation of fibers with altered properties. Unfortunately, only a few inhibitors of collagen synthesis and deposition are available, despite the importance of this protein in sustaining tissue integrity and its involvement in various disorders. Furthermore, many available inhibitors lack specificity for the collagen metabolic pathway. Thus, many currently available drugs have deleterious side effects.

For example, cytotoxic drugs have been used in an attempt to slow the proliferation of collagen-producing fibroblasts [J.A. Casas, *et al., Ann. Rhem. Dis.,* Vol. 46, p. 763 (1987)], such as colchicine, which slows collagen secretion into the extracellular matrix [D. Kershenobich, *el al., N. Engl. J. Med.,* Vol. 318, p. 1709 (1988)]. Other drugs act as inhibitors of key collagen metabolism enzymes [K. Karvonen, *et al., J Biol Chem.,* Vol. 265, p. 8414 (1990); C.J. Cunliffe, *el al., J. Med. Chem.,* Vol. 35. p.2652 (1992)]. However, none of these inhibitors have specific effects for the metabolism and deposition of specific types of collagen. Also, these drugs may interfere with the biosynthesis of other vital collagenous molecules, such as Clq in the classical complement pathway, acetylcholine esterase of the neuro-muscular junction endplate, conglutinin and pulmonary surfactant apoprotein. Such interference and lack of specificity could have potentially serious adverse effects.

Other drugs which can inhibit collagen synthesis, such as nifedipine and phenytoin, inhibit synthesis of other proteins as well, thereby non-specifically blocking the collagen biosynthetic pathway [T. Salo, *et al., J. Oral Pathol. Med,* Vol. 19, p. 404 (1990)]. Again, the lack of specificity significantly reduces the clinical use of these drugs, because the non-specific inhibition of protein synthesis can result in adverse side-effects when the drug is administered to the patient. Thus, simply preventing stricture formation alone does not make a compound useful if it is sufficiently non-specific to cause toxic side effects.

Nagler et al. (Am. J. Ostet. Gynecol., 1999, 180: 558-63) describes that Halofuginone, an inhibitor of collagen type I synthesis, prevents postoperative adhesion formation in the rat uterine horn model. WO 98/34617 discusses treatment and prevention of adhesions.

Indeed, clinically available anti-fibrotic drugs, including the collagen cross-linking inhibitors such as β-amino-propionitrile, are also non-specific. Unfortunately, the lack of specificity of these collagen cross-linking inhibitors ultimately results in severe side effects after prolonged use. These side effects include lathritic syndrome, as well as disrupted elastogenesis. The latter side effect is a result of the disruption of cross-linking of elastin, another fibrous connective tissue protein. In addition, the collagen cross-linking inhibitory effect of these drugs is secondary, so that collagen must first be overproduced before degradation by collagenase. Thus, a type-specific inhibitor of the synthesis of collagen itself is clearly required.

Such a type-specific collagen synthesis inhibitor was found by observing chickens which were fed extremely high levels of the coccidostat Halofuginone. These chickens were found to have fragile, weakened skin, as evidenced by increased skin tearing, which was caused by the inhibition of collagen synthesis [Granot, 1. *et al., Poultry Sci.,* Vol. 70, p. 1559-1563, 1991]. Hatofuginone and related compounds are disclosed in U.S. Patent No. 5,449.678 for the treatment of certain fibrotic conditions such as scleroderina and Graft Versus Host Disease. Both of these conditions are associated with excessive collagen deposition, which can be inhibited by Halofuginone. This specific inhibitor is a composition with a pharmaceutically effective amount of a pharmaceutically active compound of a formula: 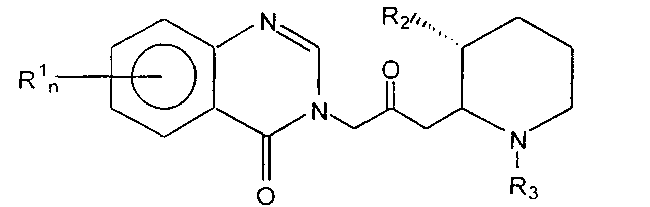 wherein:
R₁ is a member of the group consisting of hydrogen, halogen, nitro, benzo, lower alkyl, phenyl and lower alkoxy;
R₂ is a member of the group consisting of hydroxy, acetoxy and lower alkoxy;
R₃ is a member of the group consisting of hydrogen and lower alkenoxy-carbonyl; *n* is either 1 or 2; and pharmaceutically acceptable salts thereof. Of this group of compounds, Halofuginone has been found to be particularly effective for such treatment.

PCT Patent Application No. WO 96/06616 further discloses that these compounds are able to effectively treat restenosis by preventing the proliferation of vascular smooth muscle cells. Restenosis is characterized by smooth muscle cell proliferation and extracellular matrix accumulation within the lumen of affected blood vessels in response to a vascular injury [Choi *et al., Arch. Surg.,* Vol. 130, p. 257-261 (1995)]. One hallmark of such smooth muscle cell proliferation is a phenotypic alteration, from the normal contractile phenotype to a synthetic one. Type I collagen has been shown to support such a phenotypic alteration, which can be blocked by Halofuginone [Choi *et al., Arch. Surg.,* Vol. 130, p. 257-261 (1995); PCT Patent Application No. 96/06616]. Thus, Halofuginone can prevent such abnormal redifferentiation of smooth muscle cells after vascular injury by blocking the synthesis of type I collagen.

However, none of these studied models adequately predicts the behavior of Halofuginone in the promotion of wound healing or in the prevention of the formation of scars for a number of reasons.

First, the prior art teaches against the use of Halofuginone to promote wound healing, since as described above, the prior an teaches that collagen is necessary for wound healing. According to the prior art, collagen is particularly necessary for the strength of the wound. Thus, any use of Halofuginone to reduce or prevent scar formation might be expected to also prevent the healing of the wound.

Second, certainly the promotion of wound healing by Halofuginone or related compounds is neither taught nor suggested by the prior art. In fact, given the knowledge about Halofuginone and related compounds, and the expected mechanism for wound healing and scar formation, one of ordinary skill in the art would expect wound healing to be retarded in subjects receiving Halofuginone, rather than promoted. Thus, the finding that Halofuginone actually promotes wound healing without scar formation is contrary to the teachings in the art.

There is thus a widely recognized need for, and it would be highly advantageous to have, a promoter of wound healing which substantially inhibits such pathological processes as scar formation, without causing non-specific effects.

### SUMMARY OF THE INVENTION

According to the present invention, there is provided the use of a compound having a formula: 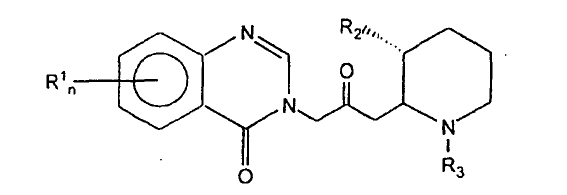 wherein:
R₁ is a member of the group consisting of hydrogen, halogen, nitro, benzo, lower alkyl, phenyl, and lower alkoxy ;
R₂ is a member of the group consisting of hydroxy, acetoxy and lower alkoxy, and R₃ is a member of the group consisting of hydrogen and lower alkenoxy-carbonyl; and n is either 1 or 2; and pharmaceutically acceptable salts thereof for the preparation of pharmaceutical composition for treating a urethral stricture in a subject, wherein the composition is to be administered locally through transurethral administration.

According to another embodiment of the invention said urethral stricture arises after a surgical procedure is performed in the subject.

According to yet another embodiment of the invention said surgical procedure is catheterization of the urethra of the subject.

According to yet another embodiment of the invention said urethral stricture arises after an infection of the urethra of the subject.

According to yet another embodiment of the invention said compound is halofuginone and pharmaceutically acceptable salts thereof.

There is provided a composition for promoting wound healing, comprising a pharmaceutically effective amount of a compound in combination with a pharmaceutically acceptable carrier, the compound being a member of a group having a formula: 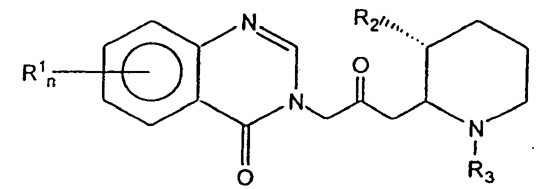 wherein:
R₁ is a member of the group consisting of hydrogen, halogen, nitro, benzo, lower alkyl, phenyl, and lower alkoxy;
R₂ is a member of the group consisting of hydroxy, acetoxy, and lower alkoxy, and
R₃ is a member of the group consisting of hydrogen and lower alkenoxy; and n is either 1 or 2; and pharmaceutically acceptable salts thereof.

There is also provided a method of manufacturing a medicament for promoting wound healing, comprising the step of placing a pharmaceutically effective amount of a compound in a pharmaceutically acceptable carrier, the compound being a member of a group having a formula: 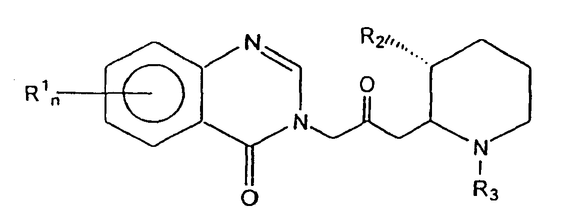 wherein:
R₁ is a member of the group consisting of hydrogen, halogen, nitro, benzo, lower alkyl, phenyl, and lower alkoxy;
R₂ is a member of the group consisting of hydroxy, acetoxy, and lower alkoxy, and
R₃ is a member of the group consisting of hydrogen and lower alkenoxy-carbonyl; and n is either 1 or 2;
and pharmaceutically acceptable salts thereof.

There is also provided a method of manufacturing a medicament for administration before a performance of a surgical procedure, for promotion of wound healing, the method comprising the step of placing a pharmaceutically effective amount of a compound in a pharmaceutically acceptable carrier, the compound being a member of a group having a formula: 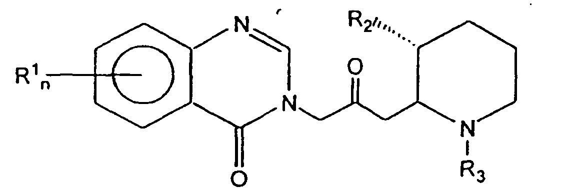 wherein:
R₁ is a member of the group consisting of hydrogen, halogen, nitro, benzo, lower alkyl, phenyl, and lower alkoxy;
R₂ is a member of the group consisting of hydroxy, acetoxy, and lower alkoxy, and
R₃ is a member of the group consisting of hydrogen and lower alkenoxy-carbonyl; and n is either 1 or 2;
and pharmaceutically acceptable salts thereof.

There is also provided a composition for treatment, substantially before a performance of a surgical procedure, for promotion of wound healing, the composition comprising a pharmaceutically effective amount of a compound having a formula: 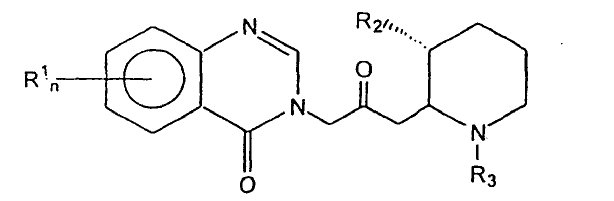 wherein:
R₁ is a member of the group consisting of hydrogen, halogen, nitro, benzo, lower alkyl, phenyl, and lower alkoxy:
R₂ is a member of the group consisting of hydroxy, acetoxy and lower alkoxy, and
R₃ is a member of the group consisting of hydrogen and lower alkenoxy-carbonyl; and *n* is either I or 2;
and pharmaceutically acceptable salts thereof.

There is also provided a composition tor treating a stricture in a subject, comprising a pharmaceutically effective amount of a compound having a formula: 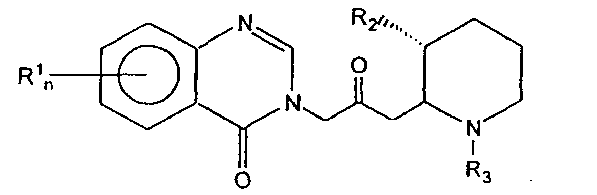 wherein:
R₁ is a member of the group consisting of hydrogen, halogen, nitro, benzo, lower alkyl, phenyl, and lower alkoxy;
R₂ is a member of the group consisting of hydroxy, acetoxy and lower alkoxy, and
R₃ is a member of the group consisting of hydrogen and lower alkenoxy-carbonyl; and n is either I or 2;
and pharmaceutically acceptable salts thereof.

Preferably, the stricture is an urethral stricture. More preferably, the urethral stricture arises after a surgerical procedure is performed in the subject. Most preferably, the surgical procedure is catheterization of the urethra of the subject.

Alternatively and preferably, the urethral stricture arises after an infection of the urethra of the subject.

According to preferred embodiments of the present invention, the compound is administered to the subject through transurethral administration for treatment of a urethral stricture.

There is provided a method for treating a stricture in a subject, comprising the step of administering to the subject a pharmaceutically effective amount of a compound having a formula: 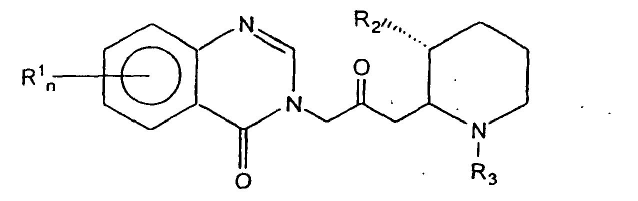 wherein:
R₁ is a member of the group consisting of hydrogen, halogen, nitro, benzo, lower alkyl, phenyl, and lower alkoxy;
R₂ is a member of the group consisting of hydroxy, acetoxy and lower alkoxy, and
R₃ is a member of the group consisting of hydrogen and lower alkenoxy-carbonyl; and n is either I or 2;
and pharmaceutically acceptable salts thereof.

According to still another embodiment of the present invention, there is provided a composition for preventing cicatrix formation in a subject while maintaining a strength of a wound, comprising a pharmaceutically effective amount of a compound having a formula: 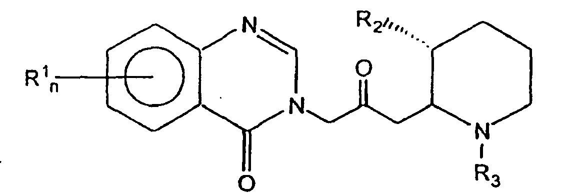 wherein: R₁ is a member of the group consisting of hydrogen, halogen, nitro, benzo, lower alkyl, phenyl, and lower alkoxy; R₂ is a member of the group consisting of hydroxy, acetoxy and lower alkoxy, and R₃ is a member of the group consisting of hydrogen and lower alkenoxy-carbonyl; and *n* is either 1 or 2; and pharmaceutically acceptable salts thereof; wherein said compound is administered to the subject, such that the strength of the wound of the subject is not decreased.

For all of these embodiments, preferably the compound is Halofuginone. Hereinafter, the term "Halofuginone" is defined as a compound having a formula: 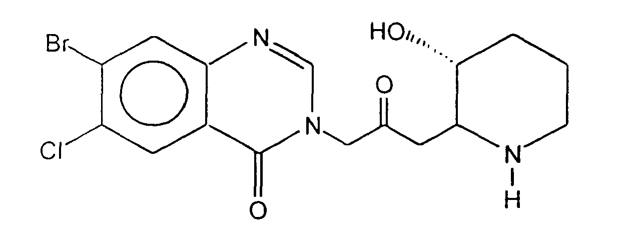 and pharmaceutically acceptable salts thereof. The composition preferably includes a pharmaceutically acceptable carrier for the compound.

Preferably, all of the compounds referred to hereinabove can be either the compound itself as described by the formula, and/or pharmaceutically acceptable salts thereof.

Hereinafter, the term "cicatrix" includes scars, strictures, hypertrophic scars, as well as substantially any other type of cicatrix. As described in greater detail below, although the examples of the efficacy of the present invention with regard to strictures are shown for urethral strictures, it is understood that this is merely an example for the sake of description and is not meant to be limiting in any way.

### BRIEF DESCRIPTION OF THE DRAWINGS

The invention is herein described, by way of example only, with reference to the accompanying drawings, wherein:
FIGS. 1A-1H illustrate the effect of Halofuginone on wound healing;
FIG. 2 illustrates the effect of Halofuginone on wound strength;
FIG. 3 illustrates the effect of Halofuginone on the promotion of wound healing after implanting a tumor;
FIG. 4 illustrates the effect of Halofuginone on the promotion of wound healing after implanting a bladder carcinoma;
FIGS. 5A-5D illustrate the effect of Halofuginone on stricture formation in the urethra;
FIG. 6 illustrates the effect of Halofuginone on collagen α1(I) gene expression in the stricture site;
FIGS. 7A-7D illustrate the effect of Halofuginone on collagen content at the stricture site;
FIGS. 8A-8C illustrate the lack of effect of Halofuginone on collagen type III at the stricture site; and
FIG. 9 illustrates the effect of Halofuginone on collagen synthesis by fibroblasts derived from rat urethra.

### DESCRIPTION OF THE PREFERRED EMBODIMENTS

Unexpectedly, compositions according to the present invention have been found to act as an inhibitor of pathological processes arising from wound healing, such as scar formation, while promoting wound healing itself. The present invention is also an inhibitor of the formation of strictures.

Specifically, the most preferred compound of the present invention, Halofuginone, can be used to inhibit scar formation, and promote wound healing, by preventing collagen deposition from occurring within the wound space. In examples detailed below, Halofuginone is shown to inhibit collagen deposition within the urethra following catheterization, thereby inhibiting the formation of urethral strictures.

In other examples given below, Halofuginone is shown to not interfere with wound heating. Such an effect is particularly unexpected because Halofuginone decreases collagen deposition. However, collagen deposition is required to strengthen the healing wound. Furthermore, high levels of Halofuginone lead to decreased skin strength and increased skin tearing. Based upon the prior art, Halofuginone would be expected to obstruct wound healing. Yet, contrary to the teachings of the prior art, Halofuginone has been specifically shown to promote wound healing, an effect which is both novel and non-obvious.

Based upon these novel, non-obvious and completely unexpected results, Halofuginone could clearly be used in a number of ways for the promotion of wound healing, as well as to maintain wound strength during the process of wound healing. For example, Halofuginone could be used to either treat formed strictures and other scars, which can be generally described as a cicatrix, such as those following surgery or inflammatory disease, or to substantially inhibit the formation of those strictures or other scars, or other examples of a cicatrix.

Halofuginone can also be used as a pretreatment, administered to a subject before surgery to substantially prevent the formation of a cicatrix. Of course, such a pretreatment would be most effective for scheduled surgery, as that would allow Halofuginone to be administered for a sufficient period of time before surgery to be most effective.

The present invention may be more readily understood with reference to the following illustrative examples and figures. It should be noted that although reference is made exclusively to Halofuginone, it is believed that the other quinazolinone derivatives described and claimed in U.S. Patent 3,320,124, have similar properties.

### Example 1

### Effect of Halofuginone on Wound Healing

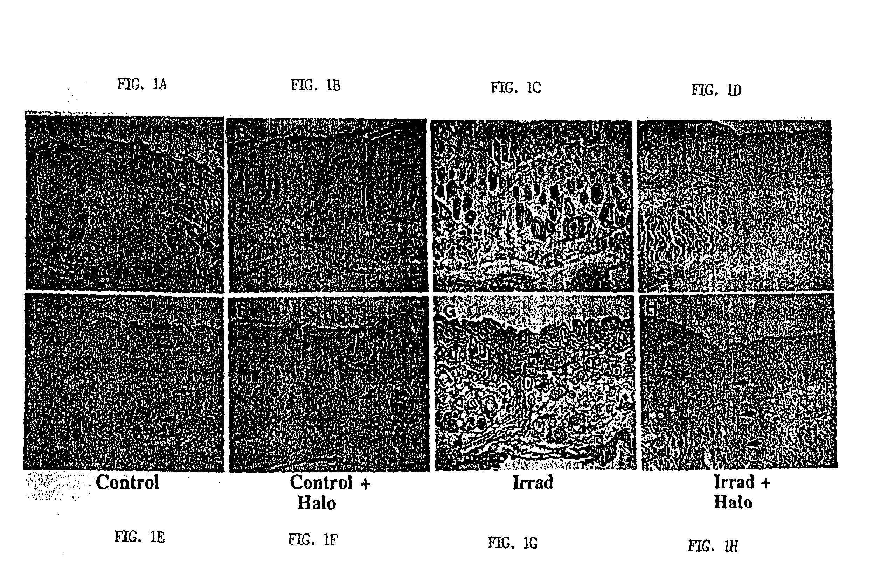

The effect of Halofuginone on wound healing was examined by using mice which were first irradiated and then wounded. As shown in Figure 1, although Halofuginone treatment caused a reduction in the collagen content of the wounded and irradiated skin, the wound still healed.

The experiment was conducted as follows. First, C3H, defined-flora, pathogen-free female mice of 12-14 weeks of age were anesthetized with 60 mg/kg sodium phenobarbital. The mice were then shaved. One group of mice was then irradiated as follows. First, a flap of skin about 40 mm long and about 20 mm wide was pulled through a slit in the lead cover of an irradiation jig and secured with tape, so that only the flap of skin was exposed. This exposed skin was then irradiated by using a 175 kVp/20 mA orthovoltage X-ray source, with a 2 mm Cu filter at a dose rate of 1.0 Gy/min. A standard dose of 18 Gy was delivered.

All of the mice were then wounded by making a full depth incision, about 25 mm long, in the skin along the midline of the lower back. Note that for the irradiated animals, the wound was made within the irradiated area, immediately following irradiation. For all mice, the incision was immediately closed by 3-4 metallic wound closure clips which were removed 2 days later.

After wounding, the mice were injected i.p. every other day, either with 1 mg per mouse of Halofuginone or with saline as a control. After 14 days, 2 days following the last injection, the mice were sacrificed and skin samples were collected into phosphate-buffered saline (PBS) and fixed in 4% paraformaldehyde in PBS at 4 °C. Serial 5 µm sections were prepared after the samples had been dehydrated in graded ethanol solutions, cleared in chloroform and embedded in Parafin. The sections were deparafinized in xylene, rehydrated through a graded series of ethanol solutions, rinsed in distilled water and treated with 0.125 mg/ml pronase in 50 mM Tris-HCl, 5 mM EDTA, pH 7.5 for 10 minutes. After digestion, slides were rinsed in distilled water, postfixed in 10% formalin in PBS, blocked in 0.2% glycine, rinsed in distilled water again, rapidly dehydrated through a graded ethanol solution and air-dried for several hours. Samples were then stained with hematoxylin-eosin (Figures 1A-D). Immunohistochemistry was performed with specific rabbit immune serum to rat collagen type I (Laboratoire de Pathologie Cellulaire. Institut Pasteur de Lyon, Lyon, France) and secondary rat anti-rabbit FITC-conjugated McAb (Figures 1E-H).

Figures 1A and 1E show tissue taken from the wound of a mouse treated with saline. Figures 1B and 1F show tissue taken from the wound of a mouse treated with Halofuginone. Figures 1C and 1G show tissue taken from the wound of an irradiated mouse treated with saline. Figures 1D and 1H show tissue taken from the wound of an irradiated mouse treated with Halofuginone. Essentially, Figure 1G shows that collagen content was higher in the wound of an irradiated mouse. However, Figure 1H shows that collagen content was significantly lowered by treatment with Halofuginone. Yet, all of these wounds healed, regardless of the collagen content.

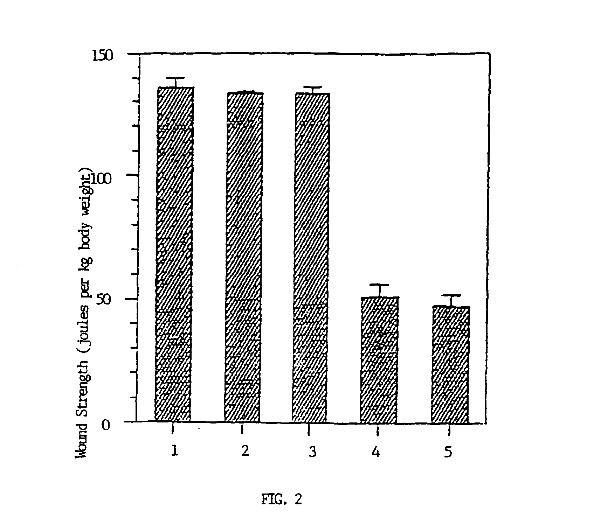

Wound strength was assessed by preparing rats substantially as described above, except that one group of rats (not irradiated) only received one injection of Halofuginone after wounding. The strength of wounds was measured as follows. First, a square of skin which was approximately 20 mm long and 16 mm wide, and which included the main part of the wound, was excised from sacrificed rats. Next, the skin was cut, perpendicular to the wound, to yield 7 strips of skin, each of which was 2 mm wide. The skin strips were secured between paper reinforcement frames and loaded on an Instron tensiometer for stretching at a constant rate of 25 mm/min. The bursting point was then recorded. Data are shown in Figure 2 for non-irradiated animals which did not receive Halofuginone (column 1), non-irradiated animals which received one (column 2) or six (column 3) injections of Halofuginone, irradiated animals which did not receive Halofuginone (column 4) and irradiated animals which received six injections of Halofuginone (column 5).

Figure 2 clearly demonstrates that Halofuginone did not reduce wound strength, whether animals were irradiated or not. However, as noted above, the prior art teaches the importance of collagen for wound strength, so that Halofuginone would be expected to reduce such wound strength. Thus, the results obtained in Figures 1 and 2 are clearly novel and non-obvious, as well as teaching against the prior art, since Halofuginone does not reduce wound strength.

### Example 2

### Halofuginone Improves Wound Healing in Mice with Tumors

Tumors from C6 rat glioma cells were prepared and implanted in nude mice. Certain mice received Halofuginone, either orally or through i.p. (intra-peritoneal) administration. The results showed that in mice which received Halofuginone, wound healing was promoted substantially without scar formation. The experimental method was as follows.

C6 rat glioma cells were cultured in DMEM supplemented with 5% FCS, 50 units/ml penicillin, 50 micrograms/ml streptomycin and 125 micrograms/ml fungizone. Aggregation of cells into small spheroids of about 150 microns was initiated by replating cells from confluent cultures onto agar-coated bacteriological plates. After 4-5 days in culture, the suspension was transferred to a 250 ml spinner flask (Bellco, USA), and the medium was changed every other day for 30-40 days. All culture operations were performed at 37 °C and 5% CO₂. Other conditions were as previously reported (Abramovitch *et al., Br. J. Cancer,* 77:440-447, 1998; Abramovitch *et al., Cancer Res.,* **55:**1956-62, 1995).

For the implantation of the cells, male CD1-nude mice, two months old and 30 g body weight, were anesthetized. A single tumor, about 1 mm in diameter, was implanted in each mouse subcutaneously in the lower back at the site of a 4 mm incision, using a Teflon tubing, as reported previously (Abramovitch *et al., Br. J. Cancer,* **77**:440-447, 1998). The incision was formed with fine surgical scissors and closed with an adhesive bandage.

The mice were then divided into six groups. One group received Halofuginone by oral administration as previously described. Four groups received different concentrations of Halofuginone through i.p. injections every other day (0.1, 0.5, 2 and 4 micrograms of Halofuginone per injection). One group received sham injections (no Halofuginone).

MRI microimaging of the implanted tumor was performed on a horizontal 4.7 T Bruker-Biospec spectrometer using an actively RF decoupled surface coil, 2 cm in diameter, and a birdcage transmission coil, as reported previously (Abramovitch *et al., Br. J. Cancer,* **77**:440-447, 1998; and Abramovitch *et al., Magn. Reson. Med.,* **39**:813-824, 1998). Mice were anesthetized and placed supine with the tumor located at the center of the surface coil. Gradient echo images were acquired with a slice thickness of 0.5 mm, TE of 10 ms, TR of 230 ms and 256x256 pixels matrix resulting in in-plane resolution of 110 microns. Growth of the capillary bed was reflected by reduction of the mean intensity at a region of interest of 1 mm surrounding the tumor (Abramovitch *et al., Br. J Cancer,* **77**:440-447, 1998; and Abramovitch *et al., Magn. Reson. Med.,* **39**:813-824, 1998).

At the end of the experimental period, the mice were sacrificed, and sections were obtained and prepared as previously described above.

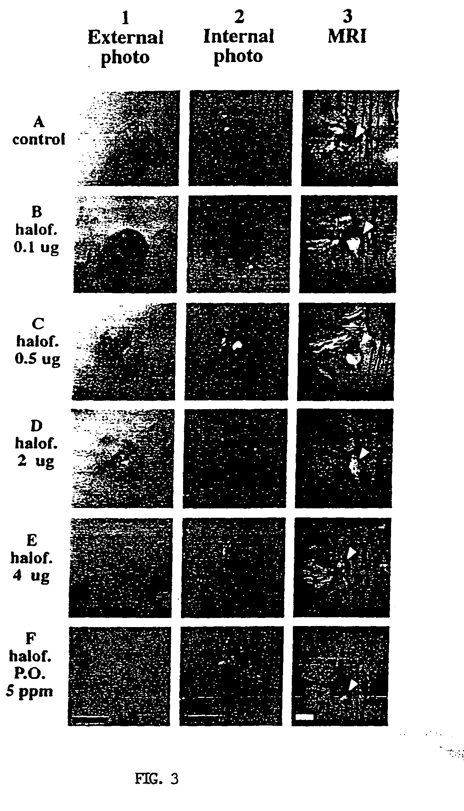

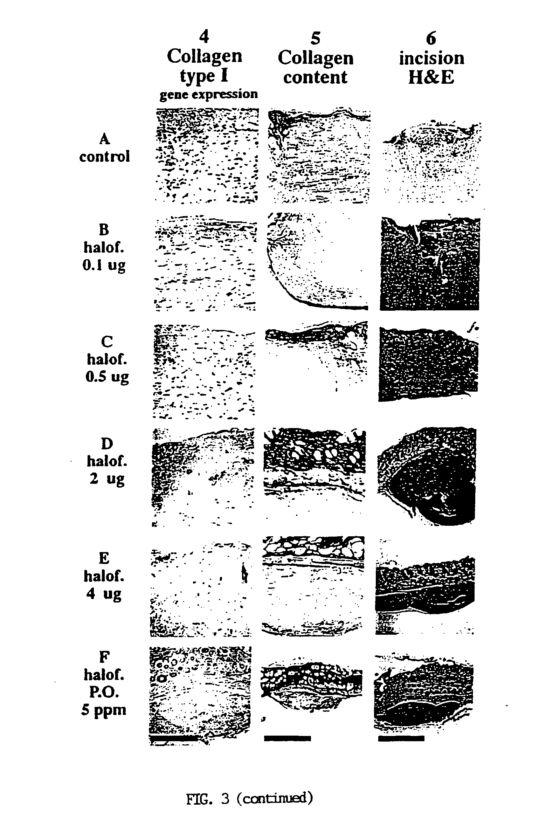

The results are shown in Figure 3. Column 1 is an external photograph of the tumor, column 2 is a photograph of the incision site, column 3 is an MRI, column 4 is collagen α1(I) gene expression, column 5 shows the collagen content and column 6 is the histology of the incision. From these results, Halofuginone significantly improved wound healing in mice, whether administered orally or as 4 micrograms per i.p. injection, despite the presence of the tumor (Figure 3), such that no scarring is found at the site of the wound. Such a result is surprising since wounds located less than 1 mm from a tumor implantation site have been previously shown not to heal rapidly. For example, re-epitheliazation was not completed in such wounds even three weeks after the incision was made, and the wound site was found to still feature inflammatory cells and blood clots (Abramovitch *et al., Br. J. Cancer,* **77**:440-447, 1998). By contrast, histological sections of the tumors demonstrated that Halofuginone induced complete re-epitheliazation in 4 out of 5 mice in each group, whether administered orally or as 4 micrograms per i.p. injection, while for all other groups, re-epitheliazation occurred in only 1 out of 5 mice in each group.

Columns 4 and 5 show that both the expression of the collagen α1(I) gene and the collagen content was decreased in rats which received Halofuginone, particularly at the highest dose (4 micrograms per i.p. injection). Thus, the decreased collagen content in mice which received Halofuginone is surprisingly accompanied by increased re-epitheliazation of the wound and the promotion of wound healing.

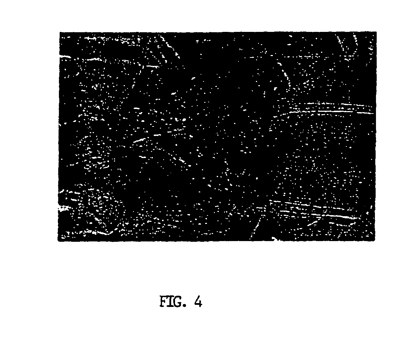

Similar visual results were obtained with mice implanted with T50 bladder carcinoma tumors (Figure 4). The experimental results were as follows. C3H mice were divided into two groups of 6 mice each. The experimental group received a diet containing either 10 mg/kg or 5 mg/kg of Halofuginone 3 days prior to the injection of T50 bladder carcinoma cells and during 2 weeks after. Cultured T50 cells, a more aggressive variant of the chemically induced MBT2 mouse bladder carcinoma, were dissociated with trypsin/EDTA into a single cell suspension (10⁶ cells/ml) in growth medium and inoculated s.c. in two sites on the dorsa of mice. The right side received 0.4 * 10⁵ cells, and the left side received 2 * 10⁵ cells. The experiment ended at day 17.

Figure 4 is a photograph of representative bladder carcinoma bearing mice which were untreated (top) or treated (bottom) with Halofuginone. Clearly, mice which were treated with Halofuginone had greater wound healing than mice which did not receive Halofuginone. Halofuginone was clearly able to promote wound healing in mice implanted with bladder carcinoma tumors. Thus, the decreased collagen content in mice which received Halofuginone is surprisingly accompanied by increased re-epitheliazation of the wound and the promotion of wound healing.

### Example 3

### Effect of Halofuginone on Stricture Formation

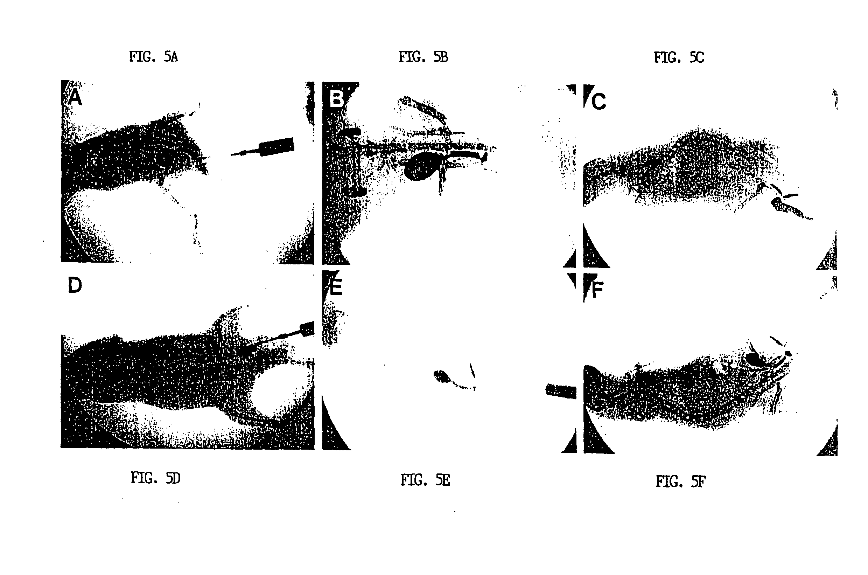

The effect of Halofuginone in post-trauma stricture formation in rats was studied. Briefly, urethral strictures were induced by catheterization and application of an electrical current to the urethra The rats were divided into four groups: untreated rats as controls (Figure 5A); rats treated with Halofuginone but not with coagulation current (Figure 5B); rats treated with coagulation current alone (Figure 5C); and rats treated with both Halofuginone and coagulation current (Figures 5D-5F). Rats which received only coagulation current had clear strictures, while rats treated with both Halofuginone and coagulation current did not have such strictures.

The experiments were performed as follows. First, urethral catherization was performed on anesthetized rats with a 23G Quik-Cath (Baxter, Ireland) sheath. A spinal needle was inserted into the sheath, and was used to apply a coagulation current at a level of 10 W to the outer end of the needle for 1 second at three locations, 8, 10 and 12 mm from the meatus, in order to produce urethral strictures. Halofuginone was given for 7 days starting at the day of stricture formation, either orally at concentrations of 1 ppm and 5 ppm in the diet, or by injection of 0.03% Halofuginone dissolved in 2% lignocaine directly into the urethra once a day. Urethral gross morphology was evaluated by urethrogram after injecting contrast material (Telebrix Megalumine 300 mg/ml, Laboratorie, Guerbet, Aulnay Sous Bios, France) into the anesthetized rat through a 23G Quik-Cath sheath, under fluoroscopy 3 weeks after stricture formation.

As shown in Figure 5C, rats treated with coagulation current alone displayed reproducible urethral strictures, accompanied by ballooning of the urethra proximal to the narrowed urethra. Also, as shown in Figure 5A, control, untreated rats, which received neither coagulation current nor Halofuginone, did not have any strictures. Furthermore, rats which received local Halofuginone with lignocaine alone, without coagulation current, also did not have any strictures, as shown in Figure 5B.

By contrast, rats which received both locally administered Halofuginone and coagulation treatment did not have strictures, nor did rats which received 5 ppm of Halofuginone in the diet with coagulation treatment (Figures 5E and 5F). The administration of only 1 ppm of Halofuginone in the diet did not affect stricture formation (Figure 5D). Thus, the administration of sufficient Halofuginone orally for 7 days after strictures were induced, or local administration of Halofuginone, was able to prevent the formation of strictures after coagulation treatment.

### Example 4

### Involvement of Collagen in Urethral Stricture Formation and the Effect of Halofuginone

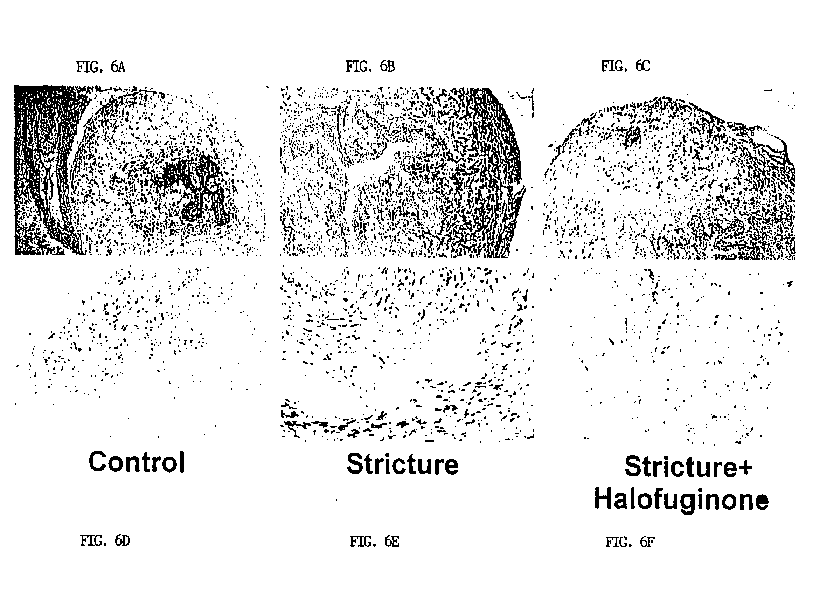

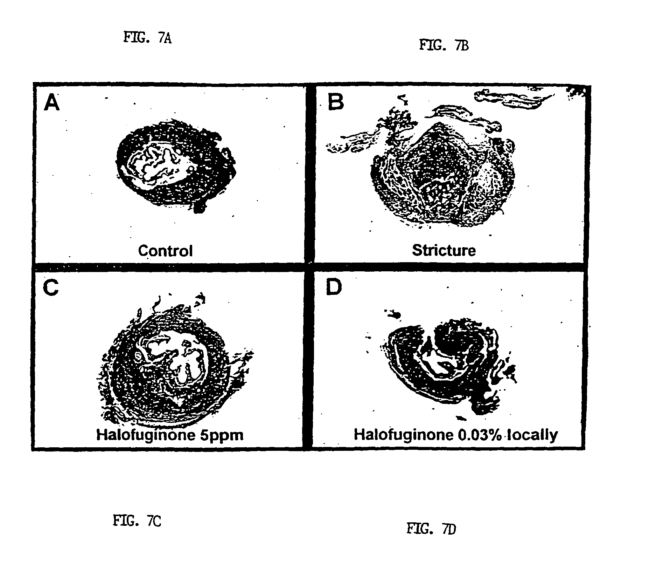

The involvement of collagen, as well as the effect of Halofuginone in post-trauma urethral stricture formation in rats was studied. Briefly, collagen-specific staining techniques, as well as hybridization with a labeled collagen-specific genetic probe, demonstrated the importance of collagen as a component of urethral strictures, as shown in Figures 6 and 7A-7D.

The experiments were performed as follows. First, urethral catherization was performed on the rats with a 23G Quik-Cath (Baxter, Ireland) sheath. A spinal needle was inserted into the sheath, and was used to apply a coagulation current at a level of 10 W to the outer end of the needle for I second at three locations, 8, 10 and 12 mm from the meatus, in order to produce urethral strictures. Halofuginone was given for 7 days starting at the day of stricture formation, either orally at concentrations of 1 ppm and 5 ppm in the diet, or by injection of 0.03% Halofuginone dissolved in 2% lignocaine directly into the urethra once a day. After 21 days, the rats were sacrificed and the sutures were reopened to determine the level of urethral stricture formation, at which time biopsies of the tissue were taken.

The tissue was sectioned so that histological studies could be performed. Briefly, the tissue samples were collected into phosphate-buffered saline (PBS) and fixed overnight in 4% paraformaldehyde in PBS at 4 °C. Serial 5 µm sections were prepared after the samples had been dehydrated in graded ethanol solutions, cleared in chloroform and embedded in Paraplast. Differential staining of collagenous and non-collagenous proteins was performed with 0.1% Sirius red and 0.1% fast green as a counter-stain in picric acid. This procedure stains collagen red [Gascon-Barre, M., *et al., J. Histochem. Cytochem.,* Vol 37, p. 377-381, 1989]. The results are shown in Figures 7A-7D.

For hybridization with the genetic probe, the sections were deparafinized in xylene, rehydrated through a graded series of ethanol solutions, rinsed in distilled water for 5 minutes and then incubated in 2X SSC at 70 °C for 30 minutes. The sections were then rinsed in distilled water and treated with pronase, 0.125 mg/ml in 50 mM Tris-HCl, 5 mM EDTA, pH 7.5, for 10 minutes. After digestion, the slides were rinsed with distilled water, post-fixed in 10% formalin in PBS and blocked in 0.2% glycine. After blocking, the slides were rinsed in distilled water, rapidly dehydrated through graded ethanol solutions and air-dried for several hours. The sections were then hybridized with a genetic probe.

Before hybridization, the genetic probe was prepared by cutting out the 1600 bp rat collagen α1(I) insert from the original plasmid, pUC18. The 1600 bp insert was then inserted into the pSafyre plasmid. The sections were then hybridized with this probe after digoxigenin-labelling. Alkaline phosphatase activity was detected in the sections as previously described [Knopov, V., *et al., Bone,* Vol 16, p. 329S-334S, 1995]. The results are shown in Figure 6.

Figure 6 shows that the cells in the biopsies taken from the urethral strictures are specifically expressing the collagen α1(I) gene, indicating the presence of collagen type I. Note that each brown dot represents a cell expressing the collagen α1(I) gene. By contrast, tissue taken from rats which received Halofuginone orally, as well as rats which did not receive coagulation treatment, had significantly reduced levels of expression of the collagen α1(I) gene. Indeed, image analysis of the results (3 biopsies for each type of treatment) demonstrated a 6.8 fold increase in the expression of the collagen α1(I) gene after treatment with coagulation treatment. By contrast, Halofuginone treatment resulted in a 5.3 fold reduction in the expression of the collagen α1(I) gene, which is an almost complete abolition of the increased expression. Thus, clearly the stricture tissue from rats which received the coagulation current alone expressed high levels of the collagen α1(I) gene, while rats which received Halofuginone and coagulation current had almost normal levels of expression.

The increased expression of the collagen α1(I) gene was accompanied by increased collagen content of the tissue, as shown in Figure 7. Figure 7B shows a section of tissue taken from one of the strictures and stained with Sirius red, while Figure 7A shows such a section of tissue taken from a control, non-treated rat. Clearly, much higher levels of staining by Sirius red are found in Figure 7B, indicating the presence of collagen in the stricture tissue. Halofuginone treatment, whether given orally (Figure 7C) or locally (Figure 7D), decreased the amount of collagen in the biopsies and prevented the lumen from filling with collagen. Thus, clearly high levels of collagen type I are present in the lumen of urethral strictures which are induced in the rats, while such high levels are abolished by the administration of Halofuginone.

### Example 5

### Halofuginone Does Not Alter Levels of Collagen Type III

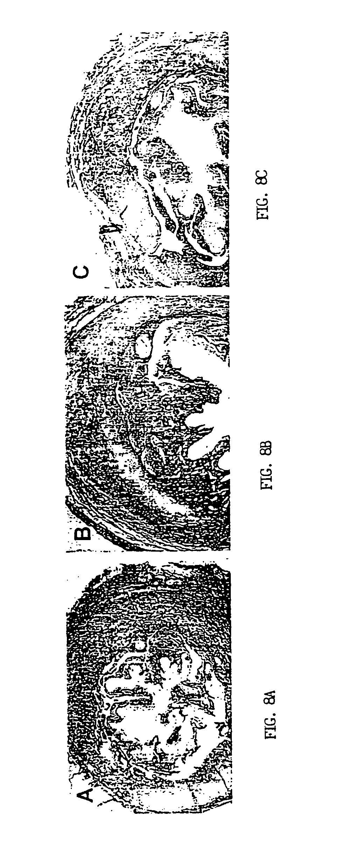

Immunohistochemistry with antibodies to collagen type III was performed on biopsies taken from control, untreated rats, rats treated with coagulation current alone and rats fed a diet containing Halofuginone after coagulation current treatment. No significant changes were observed in the levels of collagen type III between the different groups of rats (Figure 8). The experimental method was as follows.

Rats were treated and sections of biopsy tissue were prepared as described previously in Example 4. Immunohistochemistry was performed with primary antibodies against collagen type III (BioGenex, San Ramon, California, USA) and a Histomouse SP kit (Zymed Laboratories Inc., South San Francisco, California, USA) for the secondary antibodies. The primary antibodies were used in a 1:1000 dilution and detection was performed with the Histomouse SP kit according to the instructions of the manufacturer.

Figure SA shows immunohistochemistry on biopsies taken from control, untreated rats; Figure 8B shows immunohistochemistry on rats treated with coagulation current alone; and Figure SC shows immunohistochemistry on rats fed a diet containing Halofuginone after coagulation current treatment. No significant changes were observed in the levels of collagen type III between the different groups of rats. Thus, clearly the levels of collagen type III were not changed by the induction of urethral strictures or by the administration of Halofuginone.

### Example 6

### Effect of Halofuginone on Fibroblasts from Urethra

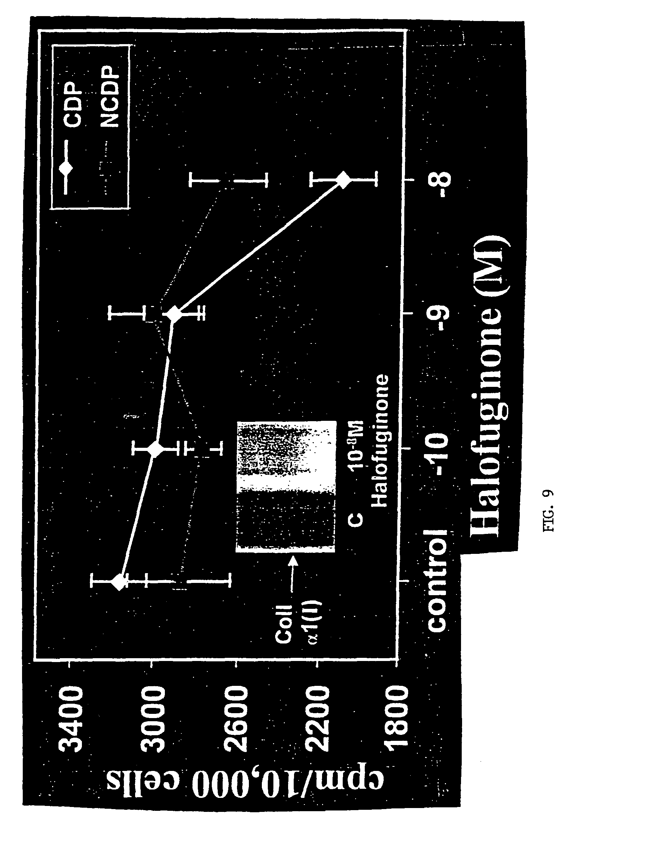

The effect of Halofuginone on fibroblasts obtained from normal rat urethra was examined. The fibroblasts were cultured from sections of urethral tissue, and then incubated in the presence or absence of Halofuginone. As shown in Figure 9, even low concentrations of Halofuginone decreased the level of collagenase-digestible proteins (CDP), while not affecting the level of non-collagenase digestible proteins (NCDP). The decreased level of CDP was shown to be caused by decreased expression of the collagen α1(I) gene. The experimental method was as follows.

Urethral fibroblasts were obtained from three normal male rats, and were processed under sterile conditions. The urethra was cut into 1-2 mm pieces and rinsed several times in a solution containing 0.2 ml/10 ml penicillin/streptomycin and 50 micrograms/ml gentamycin. Each piece of urethra was placed in a 1 ml well of a 24 well plate (Greiner Labortchnik, Germany). Each well was tilled with DMEM (Dulbecco's Modified Eagle's Medium), containing 4.5 g/ml of D-glucose. 25% FCS (fetal calf serum), 0.1 ml/10 ml penicillin/streptomycin, 0.1 ml/10 ml glutamine. 50 micrograms/ml gentamycin and 2.5 micrograms/ml amphotericin B. The wells were incubated in an incubator containing 5% CO₂ at 37 °C. Half of the medium was replaced after 10 days. Two to three days later the cultures were trypsinized with trypsin-EDTA medium. The content of each pair of wells was combined, resuspended in DMEM and placed into one 250-ml flask containing 5 ml of medium. When the cell cultures reached confluency, they were transferred to new flasks with a 1:5 ratio of old to new medium. The concentration of FCS was then decreased to 10%.

For the evaluation of the synthesis of CDP and NCDP, cells were incubated for 24 hours with various concentrations of Halofuginone in 0.5 m glutamine-free DMEM containing 5% FCS. ascorbic acid (50 micrograms/ml), β-aminopropionitrile (50 micrograms/ml) and 2 micro-curies of [³H]proline. At the end of the incubation period, the medium was decanted and incubated with or without collagenase for 18 hours, followed by TCA precipitation. The amount of radiolabelled collagen was estimated as the difference between total [³H]proline containing proteins and protein remaining after collagenase digestion. The results are shown in Figure 9.

Next, total RNA was isolated using the guanidinium-thiocyanate-phenol technique (Chomczynski, P. and N. Sacchi, *Anal. Biochem.,* **162**:156, 1987). RNA was subjected to 1% agarose denaturing gel electrophoresis, followed by blotting onto a nylon filter (GeneScreen Plus, New England Nuclear, Boston, Massachusetts, USA). The probe for the collagen α1(I) gene was labeled using the random primer procedure with a commercial kit (Boehringer, Germany). Hybridization was performed overnight at 40 °C in a solution containing 10% dextran sulfate, 1% SDS, 1 M NaCl, 40% formamide and 200 mg/ml denatured herring sperm DNA. Hybridization was followed by two 30-minute washes in 2x SSC (1x SSC contains 0.15 M NaCl and 0.015 M sodium citrate), 1% SDS, and two 20 minute washes in 1 x SSC, 0.1% SDS. The filters were exposed to X-ray film (Agfa-Curix) at -70 °C, using intensifying screens.

From the results shown in Figure 9, Halofuginone inhibited the levels of CDP by 40% even at concentrations as low as 10⁻⁸ M Halofuginone. The levels of proteins were determined from the amount of protein exported into the medium by the cultured fibroblasts, of which 52% were CDP, while the remainder were NCDP by definition. The inhibitory effect of Halofuginone did not affect the appearance of NCDP or the number of cells. The specific inhibitory effect of Halofuginone for CDP was shown to be the result of a decrease in the expression of the collagen α1(I) gene, as demonstrated by Northern blot analysis. Thus, Halofuginone specifically decreased the expression of the collagen α1(I) gene in fibroblasts taken from urethral tissue, thereby decreasing the amount of CDP while not affecting NCDP.

### Example 7

### Suitable Formulations for Administration of Halofuginone

Halofuginone can be administered to a subject in a number of ways, which are well known in the art. Hereinafter, the term "subject" refers to the human or lower animal to whom Halofuginone was administered. For example, administration may be done topically (including opthalmically, vaginally, rectally, intranasally), orally, or parenterally, for example by intravenous drip or intraperitoneal, subcutaneous, or intramuscular injection. A particularly preferred route of administration for the treatment or prevention of urethral strictures is transurethral administration.

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable. In addition, topical administration may be aided with bandages soaked in, or otherwise containing, media with the compound. The bandages can be occlusive or non-occlusive. Furthermore, a special device can be used to apply the compound. The device of the present invention includes a composition with the compound and a pharmaceutically acceptable carrier, and a container for containing the composition. Preferably, the container is a substantially sealed, sterile container, such as an aerosol-dispersing pump or a spray can. Alternatively and preferably, the container is a substantially sterile bandage. Also alternatively and preferably, the container is a squeezable tube or a gel-dispersing pump. One of ordinary skill in the art could easily select suitable containers for the composition depending upon the properties of the preferred carrier.

Compositions for oral administration include powders or granules, suspensions or solutions in water or non-aqueous media, sachets, capsules or tablets. Thickeners, diluents, flavorings, dispersing aids, emulsifiers or binders may be desirable.

Formulations for parenteral administration may include but are not limited to sterile aqueous solutions which may also contain buffers, diluents and other suitable additives.

Formulations for transurethral administration may include but are not limited to suspensions or solutions in water or non-aqueous media, optionally with a buffering compound added to the media.

Dosing is dependent on the severity of the symptoms and on the responsiveness of the subject to Halofuginone. Persons of ordinary skill in the art can easily determine optimum dosages, dosing methodologies and repetition rates.

### Example 8

### Methods of Treatment of Scars and Promotion of Wound Healing

As noted above, Halofuginone has been shown to be an effective promotor of wound healing and inhibitor of scar formation, including strictures, as well as a general inhibitor of different types of cicatrix. The following examples are illustrations only of methods of treating and preventing the formation of cicatrix such as scars, including urethral strictures, and promoting wound healing with Halofuginone, and are not intended to be limiting.

The method includes the step of administering Halofuginone, in a pharmaceutically acceptable carrier as described in Example 7 above, to a subject to be treated. Halofuginone is administered according to an effective dosing methodology, preferably until a predefined endpoint is reached, such as the absence of clinical symptoms in the subject. For example, if a subject already had a wound, the endpoint could be the reduction in size of the wound or complete healing of the wound.

Halofuginone can also be used as a pretreatment, administered to a subject before surgery to substantially prevent the formation of cicatrix such as scars and strictures, as well as promote wound healing. Of course, such a pretreatment would be most effective for scheduled surgery, as that would allow Halofuginone to be administered for a sufficient period of time before surgery to be most effective. Halofuginone would be particularly useful for cosmetic surgery, in which the inhibition of scar formation is particularly important.

Hereinafter, the term "treatment" includes both pretreatment, before a pathological condition has arisen, and treatment after the condition has arisen. For example, treatment of a wound would include both administration of Halofuginone both before and after the genesis of the wound. The term "treating" includes both treating the subject after the pathological condition has arisen, and preventing the development of the pathological condition.

### Example 9

### Method of Manufacture of a Medicament Containing Halofuginone

The following is an example of a method of manufacturing Halofuginone. First, Halofuginone is synthesized in accordance with good pharmaceutical manufacturing practice. Examples of methods of synthesizing Halofuginone, and related quinazolinone derivatives, are given in U.S. Patent No. 3,338,909. Next, Halofuginone is placed in a suitable pharmaceutical carrier, as described in Example 7 above, again in accordance with good pharmaceutical manufacturing practice.

While the invention has been described with respect to a limited number of embodiments, it will be appreciated that many variations, modifications and other applications of the invention may be made.

## Claims

1. The use of a compound having a formula: 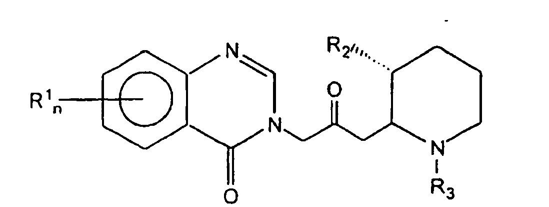 wherein:
R₁ is a member of the group consisting of hydrogen, halogen, nitro, benzo, lower alkyl, phenyl, and lower alkoxy ;
R₂ is a member of the group consisting of hydroxy, acetoxy and lower alkoxy, and R₃ is a member of the group consisting of hydrogen and lower alkenoxy-carbonyl; and *n* is either 1 or 2; and pharmaceutically acceptable salts thereof for the preparation of pharmaceutical composition for treating a urethral stricture in a subject, wherein the composition is to be administered locally through transurethral administration.

2. The use of claim 1, wherein the urethral stricture arises after a surgical procedure is performed in the subject.

3. The use of claim 2, wherein said surgical procedure is catheterization of the urethra of the subject.

4. The use of claim 1, wherein the urethral stricture arises after an infection of the urethra of the subject.

5. The use of claim 1, wherein said compound is halofuginone and pharmaceutically acceptable salts thereof.

## Patentansprüche

1. Verwendung einer Verbindung, wobei die Verbindung eine Formel 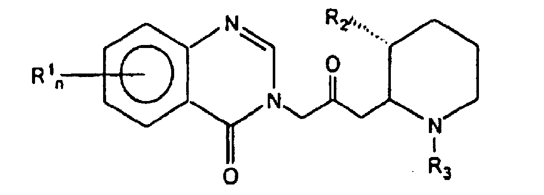 aufweist, wobei:
R₁ ein Mitglied aus der Gruppe bestehend aus Wasserstoff, Halogen, Nitro, Benzo, Niederalkyl, Phenyl und Niederalkoxy ist;
R₂ ein Mitglied aus der Gruppe bestehend aus Hydroxy, Acetoxy und Niederalkoxy ist, und R₃ ist ein Mitglied aus der Gruppe bestehend aus Wasserstoff und Niederalkenoxy-Carbonyl; und *n* ist entweder 1 oder 2; und pharmazeutisch verträgliche Salze davon zur Herstellung einer pharmazeutischen Zusammensetzung zur Behandlung einer Urethrastriktur in einer Testperson, wobei die Zusammensetzung lokal durch transurethrale Verabreichung zu verabreichen ist.

2. Die Verwendung gemäß Anspruch 1, wobei die Urethrastriktur auftritt, nachdem an der Testperson ein chirurgischer Eingriff vorgenommen wird.

3. Die Verwendung gemäß Anspruch 2, wobei dieser chirurgische Eingriff in der Katheterisierung der Urethra der Testperson besteht.

4. Die Verwendung gemäß Anspruch 1, wobei die Urethrastriktur nach einer Infektion der Urethra der Testperson auftritt.

5. Die Verwendung gemäß Anspruch 1, wobei die Verbindung Halofuginone ist, und pharmazeutisch verträgliche Salze davon.

## Revendications

1. Utilisation d'un composé ayant pour formule : 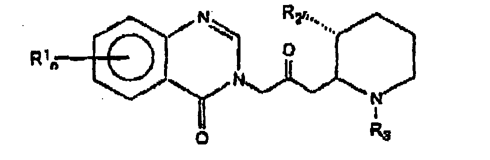 où
R₁ est un membre du groupe constitué par hydrogène, halogène, nitro, benzo, alkyle inférieur, phényle et alcoxy inférieur ;
R₂ est un membre du groupe constitué par hydroxy, acétoxy et alcoxy inférieur et R₃ est un membre du groupe constitué par hydrogène et alcènoxy-carbonyle inférieur ; et n est soit 1 soit 2 ; et leurs sels pharmaceutiquement acceptables pour la préparation d'une composition pharmaceutique destinée au traitement d'une sténose urétrale chez un sujet, où la composition doit être administrée localement par administration transurétrale.

2. Utilisation selon la revendication 1, où la sténose urétrale survient après une procédure chirurgicale réalisée chez un sujet.

3. Utilisation selon la revendication 2, où ladite procédure chirurgicale est la cathétérisation de l'urètre du sujet.

4. Utilisation selon la revendication 1, où la sténose urétrale survient après une infection de l'urètre du sujet.

5. Utilisation selon la revendication 1, où ledit composé est l'halofuginone et ses sels pharmaceutiquement acceptables.
